Europäisches Patentamt

European Patent Office

Office européen des brevets

(19)

(11) **EP 1 064 533 B1**

(12) **FASCICULE DE BREVET EUROPEEN**

(45) Date de publication et mention
de la délivrance du brevet:
**01.09.2004 Bulletin 2004/36**

(21) Numéro de dépôt: **99909062.4**

(22) Date de dépôt: **19.03.1999**

(51) Int Cl.⁷: **G01N 21/35**

(86) Numéro de dépôt international:
**PCT/FR1999/000659**

(87) Numéro de publication internationale:
**WO 1999/049299 (30.09.1999 Gazette 1999/39)**

(54) **DISPOSITIF ET PROCEDE DE MESURE DIRECTE DE L'ENERGIE CALORIFIQUE CONTENUE DANS UN GAZ COMBUSTIBLE**

VORRICHTUNG UND VERFAHREN ZUR UNMITTELBAREN MESSUNG DES HEIZWERTES EINES BRENNBAREN GASES

DEVICE AND METHOD FOR DIRECTLY MEASURING CALORIFIC ENERGY CONTAINED IN A FUEL GAS

(84) Etats contractants désignés:
**DE DK ES FR GB IT NL**

(30) Priorité: **24.03.1998 FR 9803718**

(43) Date de publication de la demande:
**03.01.2001 Bulletin 2001/01**

(73) Titulaire: **SCHLUMBERGER INDUSTRIES
92120 Montrouge (FR)**

(72) Inventeurs:
• **FROELICH, Benoit
Tokyo 145-0071 (JP)**
• **DOMINGUEZ, Didier
F-92320 Chatillon (FR)**

(74) Mandataire: **Feray, Valérie et al
Feray Lenne Conseil
44/52, Rue de la Justice
75020 Paris (FR)**

(56) Documents cités:
EP-A- 0 608 049      WO-A-93/08457
WO-A-98/32003      DE-A- 2 635 769
GB-A- 2 228 568      GB-A- 2 312 508

## Description

L'invention est relative à un dispositif de mesure de l'énergie calorifique contenue dans un gaz combustible transporté dans une conduite comprenant un compteur de gaz mesurant un volume V de gaz dans les conditions de pression P et de température T du gaz circulant dans ladite conduite et un appareil de détermination du pouvoir calorifique H du gaz.

Il est connu de mesurer l'énergie contenue dans un gaz combustible, tel que par exemple du gaz naturel, transporté dans une conduite.

Pour ce faire, un appareil tel qu'un chromatographe ou un calorimètre est généralement placé à un endroit donné de la conduite et détermine le pouvoir calorifique H du gaz sur un échantillon de gaz prélevé dans ladite conduite et ramené à des conditions dites normales de pression P0 et de température T0.

Le pouvoir calorifique H est donc déterminé dans ces conditions.

Un compteur de gaz placé à un autre endroit de la conduite, généralement en aval de l'appareil, mesure un volume V de gaz dans les conditions de pression P et de température T du gaz circulant dans ladite conduite à l'endroit où s'effectue la mesure de volume.

Par ailleurs, un appareil de correction de pression et de température et, parfois aussi de coefficient de compressibilité, est associé au compteur de gaz et transforme le volume de gaz V mesuré dans les conditions de pression P et de température T en un volume V0 ramené aux conditions dites normales de pression P0 et de température T0.

Un capteur de pression et un capteur de température sont nécessaires pour effectuer cette correction sur le volume V.

L'énergie calorifique contenue dans le gaz est alors le résultat du produit HV0.

Cette technique de mesure de l'énergie calorifique du gaz présente cependant des inconvénients car elle nécessite la mise en oeuvre d'un appareil de correction de pression et de température ainsi que des capteurs de pression et de température en sus de l'appareil de détermination du pouvoir calorifique (chromatographe, calorimètre...) qui peut être très coûteux.

Il serait par conséquent intéressant de pouvoir mesurer l'énergie calorifique contenue dans un gaz combustible de manière simplifiée par rapport à ce qui a été décrit précédemment.

Le document GB 2 312 508 décrit un dispositif de mesure de l'énergie calorifique contenue dans un gaz combustible transporté dans une conduite comprenant un compteur de gaz mesurant un volume V de gaz et un appareil de détermination du pouvoir calorifique H du gaz, ledit appareil mesurant au moins une grandeur physique proportionnelle au nombre de molécules des différents constituants du gaz et placé à proximité du compteur.

La présente invention a pour objet un dispositif de mesure de l'énergie selon la revendication 1.

Le dispositif selon l'invention permet de ne pas utiliser l'appareil de correction de pression et de température et donc de ne pas mesurer la pression et la température pour déterminer le pouvoir calorifique du gaz.

Ceci est rendu possible, d'une part, par le fait que la détermination du pouvoir calorifique s'effectue à l'endroit où le volume de gaz est mesuré et dans les mêmes conditions de température et de pression et, d'autre part, en mesurant une grandeur physique proportionnelle au nombre de molécules des différents constituants du gaz dans un volume donné.

En effet, la mesure de la grandeur physique va directement fournir le nombre de molécules présentes dans le volume donné à pression et température données.

Si la pression ou la température varient, le nombre de molécules dans le volume donné varie suivant la relation $n = PV/ZRT$, où Z désigne le coefficient de compressibilité du gaz et R est la constante des gaz parfaits, et la grandeur physique varie dans la même proportion.

Par conséquent, cette grandeur physique mesure le nombre de molécules des différents constituants du gaz indépendamment de la pression et de la température. La grandeur physique mesurée est l'absorbance d'un rayonnement électromagnétique par au moins un constituant combustible qui est présent de façon majoritaire dans le gaz et pour au moins une longueur d'onde dudit rayonnement.

L'appareil déduit ensuite le pouvoir calorifique de cette mesure d'absorbance.

Par ailleurs, le choix de cette grandeur physique est réellement intéressant puisqu'il ne nécessite pas de contact avec le gaz.

Le choix d'un rayonnement, c'est-à-dire une gamme de longueurs d'onde, pour lequel les gaz neutres ($N_2$, $O_2$, $CO_2$) n'absorbent pas est particulièrement intéressant car de tels constituants n'ont aucune contribution dans le pouvoir calorifique du gaz.

Ainsi, par exemple, les constituants $N_2$ et $O_2$ n'absorbent pas dans le rayonnement infra-rouge et le constituant $CO_2$ n'absorbe pas sur une partie du rayonnement infrarouge.

Il est donc très avantageux d'utiliser cette grandeur physique car elle permet à elle seule de s'intéresser uniquement aux constituants qui contribuent au pouvoir calorifique du gaz ce qui est donc plus simple que d'avoir plusieurs types différents de grandeurs à mesurer.

En fonction de la composition du gaz et de la précision recherchée sur le pouvoir calorifique il peut être suffisant de ne s'intéresser qu'au seul constituant combustible présent majoritairement dans le gaz, par exemple le méthane ou l'éthane ou le propane ou le butane ou le pantenne.

Afin d'accroître la précision sur la détermination du pouvoir calorique l'appareil de détermination du pouvoir calorifique du gaz mesure l'absorbance d'un rayonnement électromagnétique par d'autres constituants combustibles présents dans le gaz.

Ainsi, pour du gaz naturel, en plus de la mesure pour le constituant combustible présent de façon majoritaire dans le gaz, par exemple le méthane, on peut mesurer l'absorbance pour un ou plusieurs autres constituants combustibles minoritaires choisis parmi l'éthane, le propane, le butane et le pantenne.

La (ou les différentes) longueur(s) d'onde du rayonnement peu(ven)t être sélectionnée(s) de façon à ce qu'à chaque longueur d'onde corresponde la contribution d'un seul combustible du gaz ou de plusieurs d'entre eux.

L'appareil de détermination du pouvoir calorifique du gaz comprend, plus particulièrement, sur au moins une partie de l'écoulement du gaz :

- au moins une source d'émission du rayonnement électromagnétique à travers ladite partie d'écoulement de gaz, des moyens de filtrage dudit rayonnement,
- des moyens de détection dudit rayonnement atténué par l'absorption due au(x) constituant(s) combustible(s) du gaz, pour la ou les longueur(s) d'onde correspondante(s), fournissant un signal électrique représentatif de ce rayonnement pour chaque longueur d'onde considérée et,
- des moyens électroniques pour en déduire le pouvoir calorifique du gaz ainsi que l'énergie H (P,T) V (P,T) contenue dans le gaz.

Les moyens de filtrage du rayonnement peuvent comprendre un (ou plusieurs) filtre(s) interférentiel(s) qui est (sont chacun) adapté(s) à une longueur d'onde différente du rayonnement pour laquelle au moins l'un desdits constituants du gaz présente une absorption

Selon une autre possibilité, les moyens de filtrage du rayonnement peuvent comprendre un filtre accordable électriquement sur une gamme de longueurs d'onde incluant au moins une longueur d'onde pour laquelle ou lesquelles le(s)dit(s) constituant(s) du gaz présente(nt) une absorption.

Le rayonnement électromagnétique est par exemple situé dans infrarouge.

La gamme de longueurs d'onde inclut une (des) longueur(s) d'onde pour laquelle (lesquelles) le(s) dit(s) constituants du gaz présente(nt )une absorption est par exemple comprise entre 1 et 12µm.

Lorsque le constituant majoritaire dans le gaz est le méthane il est par exemple possible de s'intéresser à une gamme de longueurs d'onde entre 1,6 et 1,3µm.

L'invention a également pour objet un procédé de mesure de l'énergie calorifique selon la revendication 18.

Le procédé selon l'invention consiste à mesurer une grandeur physique qui est l'absorbance d'un rayonnement électromagnétique par au moins un constituant combustible présent majoritairement dans le gaz pour au moins une longueur d'onde dudit rayonnement.

Le procédé selon l'invention peut également consister à mesurer l'absorbance d'un rayonnement électromagnétique par d'autres constituants combustibles présents dans le gaz pour différentes longueurs d'onde dudit rayonnement.

Le procédé selon l'invention consiste à sélectionner la (ou les) différente(s) longueur(s) d'onde du rayonnement de façon à ce qu'à chaque longueur d'onde corresponde la contribution d'un seul constituant combustible du gaz ou de plusieurs d'entre eux.

Le procédé selon l'invention consiste, plus précisément, à :

- émettre un rayonnement électromagnétique à travers au moins une partie de l'écoulement du gaz,
- filtrer ledit rayonnement,
- détecter le rayonnement atténué par l'absorption due au(x) constituant(s) combustible(s) du gaz pour la (ou les) longueur(s) d'onde correspondante(s), et fournir un signal électrique représentatif de ce rayonnement pour chaque longueur d'onde considérée, et
- déterminer à partir du signal ou des signaux le pouvoir calorifique du gaz ainsi que l'énergie calorifique H (P,T) V (P,T) contenue dans le gaz.

Le rayonnement électromagnétique est choisi de manière à ce que les gaz neutres ($N_2$, $O_2$, $CO_2$) n'absorbent pas vis-à-vis de ce rayonnement.

Le rayonnement est par exemple choisi dans infrarouge.

D'autres caractéristiques et avantages apparaîtront au cours de la description qui va suivre donnée uniquement à titre d'exemple non limitatif et faite en référence aux dessins annexés, sur lesquels :

- la figure 1 est une vue d'ensemble d'un dispositif de mesure de l'énergie selon l'invention,

- la figure 2a représente un schéma simplifié des différents éléments constitutifs de l'appareil,

- la figure 2b représente de manière schématique des raies d'absorption distinctes pour trois longueurs d'onde différentes,

- la figure 2c représente d'une manière schématique trois spectres d'absorption A, B, C de constituants combustibles différents et leur position relative par rapport à trois longueurs d'onde différentes

- la figure 3 est une vue schématique représentant un filtre accordable électriquement,

- la figure 4 est une vue d'une variante de réalisation de l'appareil.

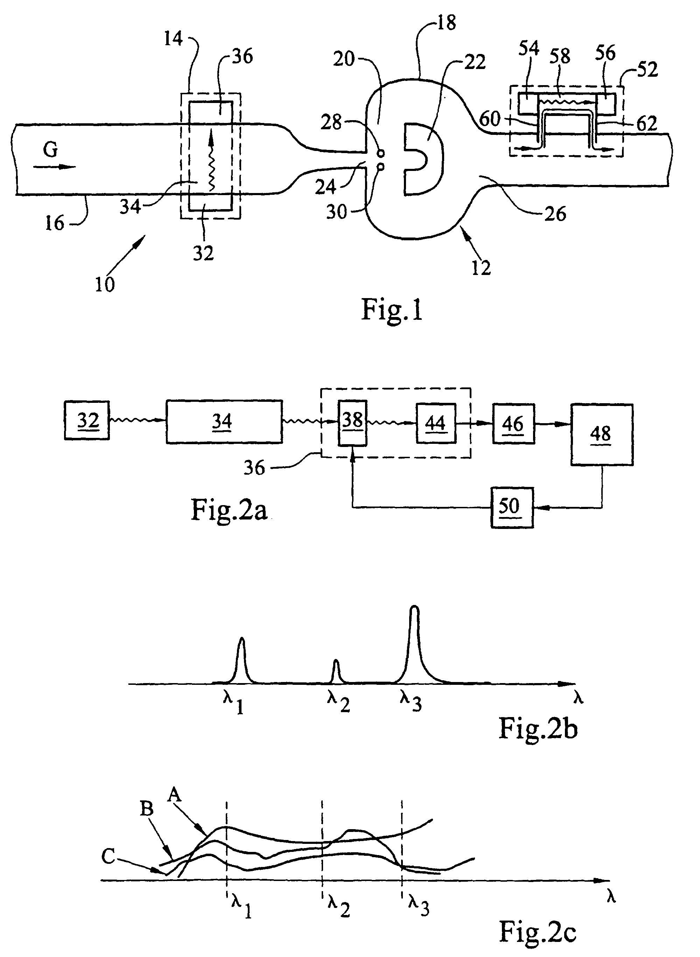

Comme représenté à la figure 1 et désigné par la référence générale notée 10, un dispositif de mesure de l'énergie contenue dans un gaz combustible, tel que par exemple du gaz naturel, comprend un compteur de gaz 12 et un appareil 14 de détermination du pouvoir calorifique H du gaz.

L'écoulement de gaz indiqué par la flèche repérée par la lettre G sur la figure 1 est transporté dans une conduite 16 où est installé le dispositif 10.

Le compteur de gaz 12 comprend une unité de mesure qui est formée d'un oscillateur fluidique de type connu et qui est décrit en détail dans la demande de brevet n°WO 97 35116.

Cet oscillateur comprend une enceinte 18 délimitant une chambre 20 dans laquelle est installé un obstacle 22 ainsi qu'une entrée 24 et une sortie 26 aménagées dans l'enceinte afin de permettre à l'écoulement de gaz respectivement d'entrer ou de sortir de ladite chambre 20.

L'entrée 24 est réalisée sous la forme d'une fente de faible largeur comparée à sa dimension longitudinale qui est perpendiculaire au plan de la figure 2, ceci afin de former un jet de gaz qui va osciller dans la chambre 20. Deux capteurs thermiques 28, 30 (représentés par des ronds sur la figure 1) permettent de détecter la fréquence d'oscillation du jet. Cet oscillateur fluidique fonctionne de la manière décrite dans le document WO 97 35116.

Il convient de noter que tout autre type de compteur de gaz pourrait être utilisé à la place de celui-ci aux fins de la présente invention.

Par exemple, un compteur de gaz volumétrique à pistons rotatifs ou à soufflets déformables ou bien encore un compteur basé sur le principe de la mesure du temps de propagation d'une onde acoustique émise dans l'écoulement entre au moins deux transducteurs acoustiques pourraient être utilisés ici.

Le compteur de gaz mesure un volume de gaz V dans les conditions de pression P et de température T qui sont celles du gaz dans la conduite 16. L'appareil 14 est disposé aussi près que possible du compteur de gaz, voire dans l'unité de mesure, si cela s'avère réalisable, afin que le pouvoir calorifique H soit directement déterminé par cet appareil dans les mêmes conditions de pression P et de température T que celles correspondant à la mesure du volume de gaz.

Ainsi, l'énergie calorifique ou enthalpie de combustion du gaz est donnée par le produit des deux mesures V (P,T) et H(P,T) sans que le volume V ait été corrigé auparavant en pression et en température. L'appareil 14 peut déterminer directement le pouvoir calorifique H(P,T) dans les conditions de pression et de température qui sont celles de l'écoulement du gaz dans la conduite car cet appareil mesure au moins une grandeur physique qui est proportionnelle au nombre de molécules des différents constituants du gaz dans un volume donné et cette mesure peut être réalisée sur les molécules soumises à la pression P et à la température T

On connaît par exemple d'après le document EP n°95308501.6 une méthode de détermination du pouvoir calorifique qui nécessite la mesure de quatre grandeurs physiques qui sont : la densité, la vitesse du son, la conductivité thermique et la viscosité.

Avantageusement, il est possible de simplifier considérablement la méthode de détermination du pouvoir calorifique en choisissant comme seule grandeur physique la propriété des constituants combustibles présents dans un gaz qui consiste à absorber un rayonnement électromagnétique pour au moins une longueur d'onde de ce rayonnement.

Dans un tel cas, le contact avec le gaz n'est pas nécessaire pour la mesure et l'appareil 14 de détermination du pouvoir calorifique peut être installé directement sur la conduite 16 sans que cela n'occasionne de perturbation sur l'écoulement du gaz.

Ainsi que représenté sur les figures 1 et 2a, cet appareil comprend une source 32 d'émission d'un rayonnement électromagnétique qui émet ce rayonnement à travers une partie 34 de l'écoulement délimitée par le faisceau de ladite source.

La source 32 est montée d'un côté de la conduite 16.

L'appareil 14 comprend, du côté opposé de la conduite, un bloc 36 qui contient, d'une part, des moyens de filtrage du rayonnement atténué par l'absorption due aux constituants combustibles du gaz et, d'autre part, des moyens de détection de ce rayonnement atténué et filtré.

Il convient de remarquer que les moyens de filtrage pourraient, à titre d'alternative, être montés du côté où se trouve la source 32.

Les moyens de filtrage peuvent comprendre un ou plusieurs filtres interférentiels classiques qui sont chacun adaptés à une longueur d'onde particulière.

Un seul filtre peut être utilisé lorsque le gaz comprend majoritairement voire uniquement du méthane et quelques gaz neutres. Le filtre est alors adapté pour filtrer le rayonnement autour d'une longueur d'onde caractéristique de l'absorption du méthane.

Toutefois, dans la plupart des cas plusieurs filtres sont prévus, soit parce que l'on désire mesurer l'absorption par d'autres constituants combustibles minoritaires en vue d'obtenir une très bonne précision sur le pouvoir calorifique, soit parce que les autres constituants combustibles présents dans le gaz contribuent de manière significative au pouvoir calorifique et leur non prise en compte se traduirait par un pouvoir calorifique totalement irréel.

Chacun des filtres est adapté à une longueur d'onde différente du rayonnement. Les longueurs d'onde, par exemple $\lambda_1$, $\lambda_2$, $\lambda_3$ peuvent être choisies de telle façon qu'à chacune d'elles correspond le spectre d'un seul constituant combustible (Fig.2b) ou bien le spectre de plusieurs constituants (Fig. 2c). Il est plus facile de choisir le premier cas car alors l'absorption par le constituant correspondant est directement obtenue par les moyens de détection.

Dans le second cas, illustré à la figure 2c par les spectres A, B et C de trois constituants combustibles différents, l'absorbance mesurée est fonction de la contribution de tous les constituants présents à la longueur d'onde considérée ($\lambda_1$, $\lambda_2$, $\lambda_3$) et il est donc nécessaire de résoudre un système d'équations où les inconnues sont les nombres de moles des différents constituants par unité de volume à la pression P et à la température T..

Il faut également veiller à ce que les longueurs d'onde soient choisies de manière à ce que pour chacune d'elles soient obtenues des informations indépendantes sur la contribution de chaque constituant.

Autrement, le système d'équations ne pourrait être résolu.

De tels filtres peuvent par exemple être installés sur un barillet monté rotatif autour d'un axe sous l'action d'un moteur.

Il est également possible de simplifier l'appareil en prévoyant à la place des filtres interférentiels un filtre 38 accordable électriquement sur une gamme de longueurs d'onde incluant la (ou les) longueur(s) d'onde pour laquelle (ou lesquelles) le (ou les) constituant(s) absorbe(nt) le rayonnement.

Le rayonnement électromagnétique choisi pourrait par exemple être situé dans le domaine des hyperfréquences ou bien dans le domaine optique. Dans le domaine optique, il pourrait s'agir de rayonnement ultraviolet ou infrarouge.

Plus particulièrement, le rayonnement optique est de type infrarouge et l'on s'intéresse précisément à une partie du spectre infrarouge sur laquelle les constituants $N_2$, $O_2$ et $CO_2$ ne présentent aucune absorption.

La source 32 est par exemple une source à large bande constituée d'un filament de tungstène.

Le filtre 38 est par exemple accordable sur la gamme de longueurs d'onde comprises entre 3,2 et 3,6µm..

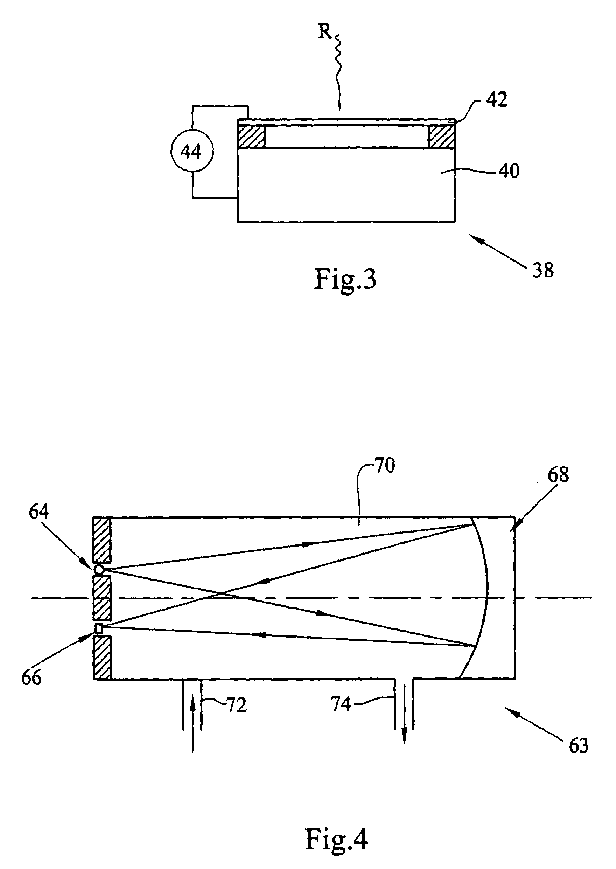

Un tel filtre est représenté à la figure 3 et est décrit en détail dans la demande de brevet européen n°0 608 049. Ce filtre, par exemple réalisé en silicium micro-usiné, est constitué d'une électrode fixe 40 formant un support et d'une électrode mobile 42 séparées l'une de l'autre d'une distance déterminée $e_0$ correspondant à une position dans laquelle l'électrode mobile n'est pas déformée.

Dans cette position dite de repos le rayonnement indiqué par la flèche repérée par la lettre R sur la figure 3 est filtré pour la longueur d'onde $\lambda_0$ qui est égale à 2 $e_0$ (et pour les harmoniques de cette longueur d'onde).

Une source de tension 44 est reliée aux électrodes mobile et fixe et, lorsqu'une tension est appliquée, l'électrode mobile se déforme et se rapproche de l'électrode fixe. La distance entre les électrodes se réduit à $e_1$ ($e_1 < e_0$) et, le rayonnement est alors filtré pour la longueur d'onde $\lambda_1$ égale à 2 $e_1$. Ainsi, pour différentes valeurs de tension électrique le filtre s'accorde sur différentes longueurs d'onde.

Comme représenté sur la figure 2, le bloc 36 comprend également un détecteur 44 à large bande tel que par exemple un bolomètre, une thermopile ou une photodiode.

L'énergie contenue dans le rayonnement infrarouge qui traverse le gaz et le filtre 38 est reçue par le détecteur 44 et est transformé en un signal électrique représentatif de ce rayonnement.

Le signal est ensuite amplifié et converti en signal numérique par le convertisseur 46 puis injecté dans un microprocesseur 48.

Un convertisseur numérique analogique 50 est utilisé pour le contrôle du filtre 38 et notamment pour sélectionner les différentes longueurs d'onde.

A titre d'exemple, le gaz est du gaz naturel dont la composition est la suivante avec le pouvoir calorifique correspondant en kilo Joule par mole :

| Méthane | 89,5 % | 891,09 |
| Ethane | 5% | 1561,13 |
| Propane | 1 % | 2220,13 |
| Butane | 0,6 % | 2873,97 |
| Pentane | 0,3 % | 3527,8 |
| Gaz neutres | 3,6 % | |

Les longueurs d'onde sont sélectionnées comme décrit précédemment et sont par exemple définies comme suit :

$\lambda_1$ = 3,2 µm
$\lambda_2$ = 3,3 µm
$\lambda_3$ = 3,4 µm
$\lambda_4$ = 3,5 µm
$\lambda_5$ = 3,6 µm

Ces longueurs d'onde sont telles qu'à chacune d'elles correspond la contribution de plusieurs constituants combustibles.

En appliquant une tension V de valeur déterminée par exemple égale à 20V au filtre 38 celui-ci s'accorde sur la longueur d'onde $\lambda_1$ et le détecteur 44 fournit un signal électrique correspondant à $S_1(V)$ :

$$S1(V) = \int_\lambda E(\lambda)\,\theta gaz(\lambda, xi)\,\theta f(\lambda, V)\,Sd(\lambda)\,d\lambda$$

où $E(\lambda)$ désigne l'intensité lumineuse émise par la source 32,
et

$$\theta gaz(\lambda, x_i) = \exp\left(-L\sum_i \alpha_i(\lambda).\,xi\right)$$

désigne la réponse spectrale due à tous les
constituants combustibles gazeux présents à cette longueur d'onde.

L désignant la longueur du trajet optique dans le gaz,
$x_i$ représentant le nombre de moles du constituant combustible i par unité de volume à la pression P et à la température T,
$\alpha_i$ désignant le coefficient d'absorption du constituant combustible i, et dépendant de la longueur d'onde, de la pression et de la température,
$\theta f(\lambda, V)$ représente la transmission optique due au filtre 38 et Sd représente la réponse spectrale du détecteur;

En accordant le filtre 38 sur les différentes longueurs d'onde $\lambda_1$ à $\lambda_5$ pour différentes valeurs de tension on mesure les valeurs $S_1(V_1)$ à $S_5(V_5)$. L'absorbance A se définit comme suit. $A(V) = Ln(1/S(V))$, où Ln désigne la fonction logarithme népérien, et l'on obtient le système suivant de cinq équations.

$$A_1(V_1) = a_{11}x_1 + a_{21}x_2 + \ldots + a_{51}x_5$$

$$A_2(V_2) = a_{12}x_1 + a_{22}x_2 + \ldots + a_{52}x_5$$

$$\ldots\ldots\ldots\ldots\ldots\ldots\ldots\ldots$$

$$A_5(V_5) = a_{15}x_1 + a_{25}x_2 + \ldots + a_{55}x_5$$

où les termes aij dépendent du constituant i et de l'appareil 14.

Avant de mettre en oeuvre l'invention sur un gaz naturel de composition non connue on procède à une étape d'étalonnage préalable en laboratoire en injectant dans l'appareil 14 plusieurs gaz avec des constituants de nombres de moles par unité de volume xi connues à T et P données.

Pour chaque gaz de composition connue, les tensions Vi (i=1,...,5) sont successivement appliquées au filtre pour que sa transmission spectrale s'accorde sur les longueurs d'onde $\lambda i(I=1,...,5)$ et pour chaque couple Vi/$\lambda i$ le détecteur fournit une valeur $Si_1(Vi)$.

Ainsi, pour le premier gaz, on obtient un système de cinq équations:

$$A_{11}(V_1)= a_{11}x_1+....+ a_{51}x_5$$

$$........................................$$

$$A_{51}(V_5)=a_{15}x_1+....+ a_{55}x_5$$

où les $x_i(i = 1, ...,5)$ sont connues et les termes $a_{ij}$ sont les inconnus.

En injectant dans l'appareil 14 quatre autres gaz de compositions connues on obtient ainsi vingt équations supplémentaires avec les mêmes termes $a_{ij}$ que précédemment.

Ceci permet alors de résoudre par une méthode mathématique connue, telle que par exemple une méthode de résolution d'équations linéaires, le système d'équations suivant où les inconnues sont les coefficients en $a_{ij}$ :

$$[A_j] = [a_{ij}][x_i],$$
$$k = 1,....,5 \qquad k = 1,....,5$$

où les indices k identifient le mélange de gaz connu concerné.

En inversant par une méthode mathématique d'inversion classique la matrice $[a_{ij}]$, on se ramène à un système

$$[x_i] = [a_{ij}]^{-1}[A_j] = [b_{ij}][A_j]$$
$$x = 1,....,5 \quad i = 1,....,5 \quad i = 1,....,5 \qquad i = 1,....,5 \quad i = 1,....,5$$
$$j = 1,....,5 \qquad j = 1,....,5$$

Ainsi, les valeurs $x_i$ s'écrivent

$$x_i = \sum_{j = 1,...,5} b_{ij} A_j(V),$$

Il suffit de mémoriser dans la mémoire du microprocesseur 48 les données $b_{ij}$ calculées lors de la calibration, et lorsqu'un gaz naturel de composition non connue et donc de pouvoir calorifique non connu est transporté dans la conduite 16, les différentes valeurs Aj(V) sont mesurées pour différentes longueurs d'onde du filtre obtenues pour les valeurs de tensions correspondantes et les termes $x_i$ s'en déduisent facilement.

Le pouvoir calorifique H (P,T) du gaz s'écrit

$$\sum_{i = 1,...,5} x_i H_i \text{ où } H_i$$

représente le pouvoir calorifique du constituant i en Joules par mole.

Par conséquent, dès que les termes xi sont déterminés, le pouvoir calorifique H (P,T) est obtenu directement.

L'énergie H (P,T) V (P,T) est alors déduite de ce qui précède.

L'appareil de détermination du pouvoir calorifique peut également par exemple être placé en aval de la chambre 20 de l'oscillateur fluidique et être désigné par la référence générale 52 (Fig.1).

Cet appareil comprend de manière analogue à l'appareil 14, une source 54, des moyens de filtrage et de détection 56 ainsi qu'une chambre 58 remplie de gaz et disposée entre la source 54 et les moyens 56.

L'appareil 52 est monté en dérivation sur la conduite 16 au moyen de deux raccords 60, 62 qui permettent respectivement d'amener une partie de l'écoulement de gaz dans la chambre 58 et de la ramener dans ladite conduite. Le raccord 60 possède une partie qui débouche dans la conduite à distance de la paroi de celle-ci afin d'échantillonner du gaz dont la composition est représentative de celle de l'écoulement.

L'installation de l'appareil 58 est avantageuse car en prévoyant deux vannes de coupure sur chacun des raccords 60, 62 il est aisé d'enlever ledit appareil, par exemple pour des raisons de maintenance, sans arrêter la circulation du gaz dans la conduite et donc en continuant à mesurer le volume de gaz V (P,T).

La figure 4 illustre une variante de l'appareil 63 de détermination du pouvoir calorifique dans laquelle la source 64, les moyens de filtrage et de détection 66 sont aménagés d'un même côté dudit appareil.

Un miroir 68 par exemple sphérique est prévu du côté opposé de l'appareil afin de réfléchir le rayonnement issu de la source sur les moyens 66. Une chambre 70 remplie de gaz est disposée entre, d'une part, le miroir 68 et, d'autre part, la source 64 et les moyens 66.

Deux raccords 72, 74 sont prévus pour amener et évacuer le gaz de la chambre 70.

Cet appareil peut être directement monté comme l'appareil 52 de la figure 1. Toutefois, il pourrait être envisagé de le placer comme l'appareil 14 en supprimant la chambre 70 et les raccords 72, 74 puisque, dans ce cas, l'écoulement de gaz de la conduite circulerait directement entre le miroir 68 et la source 64 et les moyens 66.

On pourrait également installer l'appareil à un autre endroit dans l'oscillateur fluidique lui même et, par exemple, au droit de l'entrée 24 en forme de fente.

## Revendications

**1.** Dispositif (10) de mesure de l'énergie calorifique contenue dans un gaz combustible transporté dans une conduite (16) comprenant :

- un compteur de gaz (12) mesurant un volume V de gaz dans les conditions de pression P et de température T du gaz circulant dans ladite conduite et,
- un appareil (14 ; 52 ; 63) de détermination du pouvoir calorifique H du gaz, ledit appareil de détermination du pouvoir calorifique mesurant au moins une grandeur physique proportionnelle au nombre de molécules des différents constituants du gaz dans un volume donné et étant placé à proximité du compteur de gaz,

ledit dispositif étant **caractérisé en ce que** ledit appareil comporte des moyens pour déterminer directement le pouvoir calorifique H (P,T) dans les mêmes conditions de pression P et de température T que celles dans lesquelles le volume V (P,T) de gaz est mesuré et **en ce que** ledit dispositif comporte des moyens pour déterminer ensuite l'énergie calorifique H (P,T) V (P,T) contenue dans le gaz sans que le volume V (P,T) ait été corrigé auparavant en température et en pression, ladite grandeur mesurée par ledit appareil de détermination du pouvoir calorifique H(P,T) étant l'absorbance d'un rayonnement électromagnétique par au moins un constituant combustible présent majoritairement dans le gaz pour au moins une longueur d'onde dudit rayonnement électromagnétique.

**2.** Dispositif selon la revendication 1, dans lequel l'appareil (14 ; 52 ; 63) de détermination du pouvoir calorifique H (P,T) du gaz mesure l'absorbance d'un rayonnement électromagnétique par d'autres constituants combustibles présents dans le gaz pour différentes longueurs d'onde dudit rayonnement électromagnétique.

**3.** Dispositif selon la revendication 2, dans lequel les différentes longueurs d'onde du rayonnement électromagnétique sont sélectionnées de manière à ce qu'elles correspondent chacune à la contribution d'un seul constituant combustible.

**4.** Dispositif selon la revendication 2, dans lequel à chaque longueur d'onde du rayonnement électromagnétique correspond la contribution de plusieurs constituants combustibles.

**5.** Dispositif selon l'une des revendications 1 à 4, dans lequel l'appareil de détermination du pouvoir calorifique H (P, T) du gaz comprend, sur au moins une partie de l'écoulement du gaz:

- au moins une source (32 ; 54 ; 64) d'émission du rayonnement électromagnétique à travers ladite partie d'écoulement de gaz,
- des moyens de filtrage (36 ; 38 ; 66) dudit rayonnement,
- des moyens de détection (36 ; 44 ; 66) dudit rayonnement atténué par l'absorption due au(x) constituants) combustible(s) du gaz, pour la ou les longueur(s) d'onde correspondante(s), fournissant un signal électrique représentatif de ce rayonnement pour chaque longueur d'onde considérée et,

- des moyens électroniques (18) pour en déduire le pouvoir calorifique du gaz ainsi que l'énergie H (P,T) V (P, T) contenue dans le gaz.

6. Dispositif selon la revendication 5, dans lequel tes moyens de filtrage (36 ; 38 ; 66) dudit rayonnement comprennent un (ou plusieurs) filtre(s) interférentiel(s) qui est (sont chacun) adapté(s) à une longueur d'onde différente du rayonnement pour laquelle au moins l'un desdits constituants du gaz présente une absorption.

7. Dispositif selon la revendication 5, dans lequel les moyens de filtrage dudit rayonnement comprennent un filtre (38) accordable électriquement sur une gamme de longueurs d'onde incluant au moins une longueur d'onde pour laquelle ou lesquelles le(s)dit(s) constituant(s) du gaz présente(nt) une absorption.

8. Dispositif selon l'une des revendications 1 à 7, dans lequel le rayonnement électromagnétique est choisi de manière à ce que les gaz neutres ($N_2$, $O_2$, $CO_2$) n'absorbent pas vis-à-vis de ce rayonnement.

9. Dispositif selon l'une des revendications 1 à 8, dans lequel te rayonnement optique est situé dans l'infrarouge.

10. Dispositif selon les revendications 7 et 9, dans lequel la gamme de longueurs d'onde incluant une (des) longueur(s) d'onde pour laquelle (lesquelles) le(s) dit(s) constituants du gaz présente(nt )une absorption est comprise entre 1 et 12 $\mu$m.

11. Dispositif selon l'une des revendications 1 à 10, dans lequel le méthane est utilisé comme constituant du gaz pour la détermination du pouvoir calorifique.

12. Dispositif selon l'une des revendications 1 à 11, dans lequel l'éthane est utilisé comme constituant du gaz pour la détermination du pouvoir calorifique.

13. Dispositif selon l'une des revendications 1 à 12, dans lequel le propane est utilisé comme constituant du gaz pour la détermination du pouvoir calorifique.

14. Dispositif selon l'une des revendications 1 à 13, dans lequel le butane est utilisé comme constituant du gaz pour la détermination du pouvoir calorifique.

15. Dispositif selon l'une des revendications 1 à 14, dans lequel le pentane est utilisé comme constituant du gaz pour la détermination du pouvoir calorifique.

16. Dispositif selon l'une des revendications 1 à 15, dans lequel le gaz combustible est du gaz naturel.

17. Dispositif selon l'une des revendications 1 à 16, dans lequel l'appareil (14 ; 52; 63) de détermination du pouvoir calorifique H (P,T) du gaz est intégré au compteur de gaz (12).

18. Procédé de mesure de l'énergie calorifique contenue dans un gaz combustible transporté dans une conduite (16) consistant à :

- mesurer un volume V de gaz dans les conditions de pression P et de température T du gaz circulant dans ladite conduite et,
- déterminer le pouvoir calorifique H du gaz,

ledit procédé consistant à mesurer dans les conditions de pression P et de température T qui sont celles dans lesquelles le volume V (P, T) de gaz est mesuré, au moins une grandeur physique proportionnelle au nombre de molécules des différents constituants du gaz dans un volume donné de gaz,
ledit procédé étant **caractérisé en ce qu'**il comporte les étapes de :

- détermination directe du pouvoir calorifique H (P,T) à partir de ladite mesure d'au moins une grandeur physique, dans les mêmes conditions de pression P et de température T,
- détermination de l'énergie calorifique H (P,T) V (P,T) contenue dans le gaz sans que le volume V (P,T) ait été corrigé auparavant en température et en pression,

**EP 1 064 533 B1**

ladite grandeur mesurée étant l'absorbance d'un rayonnement électromagnétique par au moins un constituant combustible présent majoritairement dans le gaz pour au moins une longueur d'onde dudit rayonnement électromagnétique.

19. Procédé selon la revendication 18, consistant à mesurer l'absorbance d'un rayonnement électromagnétique par d'autres constituants combustibles présents dans le gaz pour différentes longueurs d'onde dudit rayonnement.

20. Procédé selon la revendication 19, consistant à sélectionner la (ou les) longueur(s) d'onde du rayonnement électromagnétique de manière à ce qu'à chacune d'elles corresponde la contribution d'un seul constituant combustible.

21. Procédé selon la revendication 19, consistant à sélectionner la (ou les) longueur(s) d'onde du rayonnement électromagnétique de manière à ce qu'à chacune d'elles corresponde la contribution de plusieurs constituants combustibles.

22. Procédé selon l'une des revendications 18 à 21, consistant à:

   - émettre un rayonnement électromagnétique à travers au moins une partie de l'écoulement du gaz,
   - filtrer ledit rayonnement,
   - détecter le rayonnement atténué par l'absorption due au(x) constituant(s) combustible(s) du gaz pour la (ou les) longueur(s) d'onde correspondante(s), et fournir un signal électrique représentatif de ce rayonnement pour chaque longueur d'onde considérée, et
   - déterminer à partir du signal ou des signaux le pouvoir calorifique du gaz ainsi que l'énergie H (P,T) V (P,T) contenue dans le gaz.

23. Procédé selon l'une des revendications 18 à 22, selon lequel le rayonnement électromagnétique est choisi de manière à ce que les gaz neutres ($N_2$, $O_2$, $CO_2$) n'absorbent pas vis-à-vis de ce rayonnement.

24. Procédé selon l'une des revendications 18 à 23, selon lequel le rayonnement optique est situé dans l'infra-rouge.

**Patentansprüche**

1. Vorrichtung (10) zur Messung der Wärmeenergie, die in einem durch eine Leitung (16) transportierten, brennbaren Gas enthalten ist, mit:

   - einem Gaszähler (12), der bei den Bedingungen für Druck P und Temperatur T ein Gasvolumen V des in der Leitung zirkulierenden Gases misst, und
   - einer Einrichtung (14; 52; 63) zur Bestimmung des Brennwerts H des Gases, wobei die Bestimmungseinrichtung für den Brennwert zumindest eine physikalische Größe misst, die proportional zur Anzahl von Molekülen der unterschiedlichen Bestandteile des Gases in einem gegebenen Volumen ist, und in der Nähe des Gaszählers platziert ist,

   **dadurch gekennzeichnet, dass** die Einrichtung Mittel enthält, um direkt den Brennwert H(P,T) unter den Bedingungen für Druck P und Temperatur T zu bestimmen, die gleich denjenigen sind, unter denen das Gasvolumen V(P,T) gemessen wird, und dass die Vorrichtung Mittel umfasst, dann die in dem Gas enthaltene Wärmeenergie H(P,T)V(P,T) zu bestimmen, ohne dass das Volumen V(P,T) zuvor für Temperatur und Druck korrigiert wurde, wobei die durch die Bestimmungseinrichtung für den Brennwert H(P,T) gemessene Größe der Absorptionsgrad einer elektromagnetischen Strahlung durch zumindest einen in dem Gas vorherrschend vorhandenen, brennbaren Bestandteil für zumindest eine Wellenlänge der elektromagnetischen Strahlung ist.

2. Vorrichtung nach Anspruch 1, wobei die Bestimmungseinrichtung (14; 52; 63) für den Brennwert H(P,T) des Gases den Absorptionsgrad einer elektromagnetischen Strahlung durch weitere in dem Gas vorhandene brennbare Bestandteile für andere Wellenlängen der elektromagnetischen Strahlung misst.

3. Vorrichtung nach Anspruch 2, wobei die verschiedenen Wellenlängen der elektromagnetischen Strahlung so ausgewählt werden, dass sie jeweils dem Beitrag eines einzigen brennbaren Bestandteils entsprechen.

4. Vorrichtung nach Anspruch 2, wobei jede Wellenlänge der elektromagnetischen Strahlung dem Beitrag mehrerer brennbarer Bestandteile entspricht.

5. Vorrichtung nach einem der Ansprüche 1 bis 4, wobei die Bestimmungseinrichtung für den Brennwert H(P,T) des Gases auf zumindest einem Abschnitt der Gasströmung folgendes umfasst:

- zumindest eine Quelle (32; 54; 64) zur Emission der elektromagnetischen Strahlung durch den Gasströmungsabschnitt,
- Filtermittel (36; 38; 66) für die Strahlung;
- Erfassungsmittel (36; 44; 66) für die durch Absorption aufgrund des (der) brennbaren Bestandteils(e) des Gases für die entsprechende(n) Wellenlänge(n) geschwächte Strahlung, die ein für diese Strahlung repräsentatives Signal für jede betreffende Wellenlänge liefern, und
- elektronische Mittel (18), um daraus den Brennwert des Gases sowie die in dem Gas enthaltenen Energie H(P,T)V(P,T) abzuleiten.

6. Vorrichtung nach Anspruch 5, wobei die Filtermittel (36; 38; 66) für die Strahlung ein (oder mehrere) Interferenzfilter umfassen, das (die) jeweils für eine andere Wellenlänge der Strahlung geeignet ist (sind), für die zumindest einer der Bestandteile des Gases eine Absorption aufweist.

7. Vorrichtung nach Anspruch 5, wobei die Filtermittel für die Strahlung ein Filter (38) umfassen, das elektrisch auf einen Wellenlängenbereich abstimmbar ist, der zumindest eine Wellenlänge enthält, für die der (die) Bestandteil(e) des Gases eine Absorption aufweist(en).

8. Vorrichtung nach einem der Ansprüche 1 bis 7, wobei die elektromagnetische Strahlung so gewählt wird, dass die Neutralgase ($N_2$, $O_2$, $CO_2$) diese Strahlung nicht absorbieren.

9. Vorrichtung nach einem der Ansprüche 1 bis 8, wobei die optische Strahlung im Infraroten liegt.

10. Vorrichtung nach einem der Ansprüche 7 und 9, wobei der Wellenlängenbereich, der eine (mehrere) Wellenlänge(n) enthält, für die der (die) Bestandteil(e) des Gases aufweist eine Absorption(en), die zwischen 1 und 12 µm liegt.

11. Vorrichtung nach einem der Ansprüche 1 bis 10, wobei Methan als Bestandteil des Gases für die Bestimmung des Brennwerts verwendet wird.

12. Vorrichtung nach einem der Ansprüche 1 bis 11, wobei Ethan als Bestandteil des Gases für die Bestimmung des Brennwerts verwendet wird.

13. Vorrichtung nach einem der Ansprüche 1 bis 12, wobei Propan als Bestandteil des Gases für die Bestimmung des Brennwerts verwendet wird.

14. Vorrichtung nach einem der Ansprüche 1 bis 13, wobei Butan als Bestandteil des Gases für die Bestimmung des Brennwerts verwendet wird.

15. Vorrichtung nach einem der Ansprüche 1 bis 14, wobei Pentan als Bestandteil des Gases für die Bestimmung des Brennwerts verwendet wird.

16. Vorrichtung nach einem der Ansprüche 1 bis 15, wobei das brennbare Gas Erdgas ist.

17. Vorrichtung nach einem der Ansprüche 1 bis 16, wobei die Einrichtung (14; 52; 63) zur Bestimmung des Brennwerts H(P,T) des Gases in den Gaszähler (12) integriert ist.

18. Verfahren zum Messen der Wärmeenergie, die in einem durch eine Leitung (16) transportierten, brennbaren Gas enthalten ist, das darin besteht:

- bei den Bedingungen für Druck P und Temperatur T ein Gasvolumen V des in der Leitung zirkulierenden Gases zu messen,
- den Brennwerts H des Gases zu bestimmen,

  wobei das Verfahren darin besteht, bei den Bedingungen für Druck P und Temperatur T, die diejenigen sind, bei denen das Gasvolumen V(P,T) gemessen wird, zumindest eine physikalische Größe zu messen, die proportional zur Anzahl von Molekülen der unterschiedlichen Bestandteile des Gases in einem gegebenen Volumen ist, und in der Nähe des Gaszählers platziert ist,

wobei das Verfahren **dadurch gekennzeichnet ist, dass** es die folgenden Schritte umfasst:

- direkte Bestimmung des Brennwerts H(P,T) unter den gleichen Bedingungen für Druck P und Temperatur T auf der Basis der Messung zumindest einer physikalischen Größe,
- Bestimmung der in dem Gas enthaltene Wärmeenergie H(P,T)V(P,T), ohne dass das Volumen V(P,T) zuvor für Temperatur und Druck korrigiert wurde,

wobei die gemessene Größe der Absorptionsgrad einer elektromagnetischen Strahlung durch zumindest einen in dem Gas vorherrschend vorhandenen, brennbaren Bestandteil für zumindest eine Wellenlänge der elektromagnetischen Strahlung ist.

19. Verfahren nach Anspruch 18, das darin besteht, den Absorptionsgrad einer elektromagnetischen Strahlung durch weitere, in dem Gas vorhandene, brennbare Bestandteile für unterschiedliche Wellenlängen der Strahlung zu messen.

20. Verfahren nach Anspruch 19, das darin besteht, die wellenlänge(n) der elektromagnetischen Strahlung so auszuwählen, dass jeder von ihnen der Beitrag eines einzigen brennbaren Bestandteils entspricht.

21. Verfahren nach Anspruch 19, das darin besteht, die Wellenlänge(n) der elektromagnetischen Strahlung so auszuwählen, dass jeder von ihnen der Beitrag von mehreren brennbaren Bestandteilen entspricht.

22. Verfahren nach einem der Ansprüche 18 bis 21, das darin besteht:

- eine elektromagnetische Strahlung durch zumindest einen Teil des Gasstroms zu emittieren,
- die Strahlung zu filtern,
- die durch die Absorption aufgrund des (der) brennbaren Bestandteils(e) für die entsprechende(n) Wellenlänge(n) geschwächte Strahlung zu erfassen und ein für diese Strahlung repräsentatives elektrisches Signal für jede betreffende Wellenlänge zu liefern, und
- auf der Basis des Signals oder der Signale den Brennwert des Gases sowie die in dem Gas enthaltene Energie H(P,T)V(P,T) zu bestimmen.

23. Verfahren nach einem der Ansprüche 18 bis 22, wobei die elektromagnetische Strahlung so gewählt wird, dass die Neutralgase ($N_2$, $O_2$, $CO_2$) diese Strahlung nicht absorbieren.

24. Verfahren nach einem der Ansprüche 18 bis 23, wobei die optische Strahlung im Infraroten liegt.

## Claims

1. Device (10) for measuring the calorific energy contained in a combustible gas transported in a pipe (16), the device comprising:

- a gas meter (12) for measuring a volume V of gas under the conditions of pressure P and temperature T of the gas flowing in said pipe, and
- an apparatus (14; 52; 63) for determining the gross calorific value H of the gas, said apparatus measuring at least one physical parameter proportional to the number of molecules of the constituents of the gas in a given volume and being disposed near the gas meter, said device being **characterized in that** said apparatus comprises means for determining directly the gross calorific value H(P,T) under the same conditions of pressure P and temperature T as those under which the volume V(P,T) of gas is measured and **in that** said device comprises means for then determining the calorific energy H(P,T) V(P,T) contained in the gas without the volume V(P,T) having been corrected beforehand for temperature and pressure, said parameter measured by said apparatus for determining the gross calorific value H(P,T) being the absorbance of electromagnetic radiation by at least one majority combustible constituent present in the gas at one wavelength at least of said electromagnetic radiation.

**2.** Device according to claim 1, wherein the apparatus (14;52;63) for determining the gross calorific value H(P,T) of the gas measures the absorbance of electromagnetic radiation by other combustible constituents present in the gas for different wavelengths of said electromagnetic radiation.

**3.** Device according to claim 2, wherein the wavelengths of the electromagnetic radiation are selected so that each corresponds to the contribution of only one combustible constituent.

**4.** Device according to claim 2, wherein each wavelength of the electromagnetic radiation corresponds to the contribution of a plurality of combustible constituents.

**5.** Device according to any of claims 1 to 4, wherein the apparatus for determining the gross calorific value H(P,T) of the gas comprises, over at least a portion of the flow of the gas:

- at least one source (32; 54; 64) for emitting electromagnetic radiation into said gas flow portion,
- means (36; 38; 66) for filtering said radiation,
- means (36; 44; 66) for detecting said radiation when attenuated by absorption by the combustible constituent or constituents of the gas, for the corresponding wavelength or wavelengths, and supplying an electrical signal representative of that radiation for the wavelength or each wavelength concerned, and
- electronic means (18) for deducing the gross calorific value of the gas and the energy H(P,T) V(P,T) contained in the gas.

**6.** Device according to claim 5, wherein the means (36; 38; 66) for filtering said radiation comprise one or more interference filters adapted to a different wavelength of the radiation which is absorbed by at least one of said constituents of the gas.

**7.** Device according to claim 5, wherein the means for filtering said radiation comprise a filter (38) that may be tuned electrically over a range of wavelengths including at least one wavelength absorbed by said constituent or constituents of the gas.

**8.** Device according to any of claims 1 to 7, wherein the electromagnetic radiation is chosen so that neutral gases ($N_2$, $O_2$, $CO_2$) do not absorb the radiation.

**9.** Device according to any of claims 1 to 8, wherein the optical radiation is infrared radiation.

**10.** Device according to claims 7 and 9, wherein the range of wavelengths including one or more wavelengths absorbed by said constituent or constituents of the gas is from 1 $\mu$m to 12$\mu$m.

**11.** Device according to any of claims 1 to 10, wherein methane is the constituent of the gas used for determining the gross calorific value.

**12.** Device according to any of claims 1 to 11, wherein ethane is the constituent of the gas used for determining the gross calorific value.

**13.** Device according to any of claims 1 to 12, wherein propane is the constituent of the gas used for determining the gross calorific value.

**14.** Device according to any of claims 1 to 13, wherein butane is the constituent of the gas used for determining the gross calorific value.

**15.** Device according to any of claims 1 to 14, wherein pentane is the constituent of the gas used for determining the gross calorific value.

**16.** Device according to any of claims 1 to 15, wherein the combustible gas is natural gas.

**17.** Device according to any of claims 1 to 16, wherein the apparatus (14; 52; 63) for determining the gross calorific value H(P,T) of the gas is integrated into the gas meter (12).

**18.** A method of measuring the calorific energy contained in a combustible gas transported in a pipe (16), the method consisting in:

- measuring a volume V of gas under the same conditions of pressure P and temperature T as the gas flowing in said pipe, and
- determining the gross calorific value H of the gas,

said method consisting in measuring under the same conditions of pressure P and temperature T as those under which the volume V(P,T) of gas is measured at least one physical parameter proportional to the number of molecules of the constituents of the gas in a given volume of gas,
said method being **characterized in that** it includes the steps of:

- direct determination of the gross calorific value H(P,T) from said measurement of at least one physical parameter under the same conditions of pressure P and temperature T,
- determination of the calorific energy H(P,T) V(P,T) contained in the gas without the volume V(P,T) having to be corrected beforehand for temperature and pressure,

said measured parameter being the absorbance of electromagnetic radiation by at least one majority combustible constituent present in the gas for at least one wavelength of said electromagnetic radiation.

**19.** Method according to claim 18, consisting in measuring the absorbance of electromagnetic radiation by other combustible constituents present in the gas for different wavelengths of said radiation.

**20.** Method according to claim 19, consisting in selecting the wavelength or wavelengths of the electromagnetic radiation so that the wavelength or each wavelength corresponds to the contribution of only one combustible constituent.

**21.** Method according to claim 19, consisting in selecting the wavelength or wavelengths of the electromagnetic radiation so that the wavelength or each wavelength corresponds to the contribution of a plurality of combustible constituents.

**22.** Method according to any of claims 18 to 21, consisting in:

- emitting electromagnetic radiation into at least a portion of the flow of the gas,
- filtering said radiation,
- detecting the radiation attenuated by absorption by the combustible constituent or constituents of the gas for the corresponding wavelength or wavelengths, and supplying an electrical signal representative of said radiation for the wavelength or each wavelength concerned, and
- determining the gross calorific value of the gas and the energy H(P,T) V(P,T) contained in the gas from the electrical signal or signals.

**23.** Method according to any of claims 18 to 22, wherein the electromagnetic radiation is chosen so that neutral gases ($N_2$, $O_2$ $CO_2$) do not absorb the radiation.

**24.** Method according to any of claims 18 to 23, wherein the optical radiation is infrared radiation.

Fig.1

Fig.2a

Fig.2b

Fig.2c

R

42

44

40

38

Fig.3

70

68

64

66

72    74

63

Fig.4